# EUROPEAN PATENT SPECIFICATION

(11) **EP 0 966 302 B1**
(45) Date of publication and mention of the grant of the patent: **14.03.2007**
(21) Application number: 98901227.3
(22) Date of filing: 09.01.1998
(51) Int. Cl.: A61K 39/395

(54) **METHODS OF THERAPEUTIC ADMINISTRATION OF ANTI-CD40L COMPOUNDS**
VERFAHREN ZUR THERAPEUTISCHEN VERABREICHUNG VON ANTI-CD40L-MITTELN
PROCEDES D'ADMINISTRATION THERAPEUTIQUE DE COMPOSES ANTI-CD40L

(30) Priority: 10.01.1997 US 34672 P; 07.03.1997 US 40154 P
(43) Date of publication of application: 29.12.1999
(73) Proprietor: Biogen Idec MA Inc., Cambridge, Massachusetts 02142 (US)
(72) Inventor: KALLED, Susan, L., Jamaica Plain, MA 02130 (US); THOMAS, David, W., Wellesley, MA 02182 (US)
(74) Representative: UEXKÜLL & STOLBERG
(86) International application number: PCT/US1998/000573
(87) International publication number: WO 1998/030241

(56) References cited:
- WO-A-93/09812
- WO-A-95/06480
- WO-A-96/23071
- F. DURIE ET AL.: "Prevention of collagen-induced arthritis with an antibody to gp39, the ligand for CD40." SCIENCE, vol. 261, no. 5126, 3 September 1993, WASHINGTON, DC, USA, pages 1328-1330, XP002064747 cited in the application
- K. GERRITSE ET AL.: "CD40-CD40 ligand interactions in experimental allergic encephalomyelitis and multiple sclerosis." PROCEEDINGS OF THE NATIONAL ACADEMY OF SCIENCES OF THE USA, vol. 93, no. 6, 19 March 1996, WASHINGTON,DC, USA, pages 2499-2504, XP002064748
- G. EARLY ET AL.: "Anti-CD40 ligand antibody treatment prevents the development of lupus-like nephritis in a subset of New Zealand black x New Zealand white mice." THE JOURNAL OF IMMUNOLOGY, vol. 157, no. 7, 1 October 1996, BALTIMORE, MD, USA, pages 3159-3164, XP002064749
- Stüber, E. et al (1996) J. Exp. Med. 183:693-698

## Description

### Field of the Invention

The invention relates to the use of an anti-CD40L antibody for the manufacture of a medicament for treating a patient with systemic lupus erythematosus.

### Background of the Invention

One of the necessary reactions in the generation of antibodies is the interaction of CD40 on B cells with CD40 ligand (CD40L) on activated T cells, a step which is required for B cell growth and subsequent production of antibodies. (Note: "gp39" and "T Cell-B Cell Activating Molecule" (T-BAM) are used synonymously for CD40L in some reports.) It is understood that CD40L, gp39 and T-BAM are one and the same molecule. As further described below, a number of anti-CD40L compounds have been produced, and some have been tested in animals for efficacy in altering the course of antibody-associated diseases. For example, PCT Publication WO 93/09812 (27 May 1993) discloses a murine monoclonal antibody, designated 5c8, which binds specifically to T-BAM on the surface of activated T cells thereby inhibiting Tcell activation of B cells. The protocols used in the reported experiments on effects of anti-CD40L compounds on animals with immune disorders have employed doses of the compounds administered to the animals at intervals of two weeks or less, with typical intervals between treatments being 1-7 days. (See, e.g., Mohan et al., J. Immunol. 154: 1470-1480, 1995; Early et al., J. Immunol. 157: 3159-3164, 1996; Stüber et al., J. Exp. Med. 183:693-698, 1996; Chen et al., J. Immunol. 155:2833-2840, 1995; Gerritse et al., Proc. Natl. Acad. Sci. 93:2499-2504, 1996; Green et al., T. Virol. 70:2569-2575, 1996; Durie et al., Science 261:1328-1330, 1993; Durie et al., J. Clin. Invest. 94:1333-1338, 1994; Larsen et al., Transplantation 61:4-9, 1996; and Griggs et al., J. Exp. Med 183:801-810, 1996). There has been no available information that suggests that less frequent administration of anti-CD40L compounds would be efficacious in inhibiting the production of pathologic antibodies or improving the course of immune-related diseases.

### Summary of the Invention

The invention provides the use of an anti-CD40L antibody or an anti-CD40L antibody derivative for the manufacture of a medicament for treating a human patient with systemic lupus erythematosus, wherein
a therapeutically effective amount of said antibody or antibody derivative is administered to the patient for a first therapeutic period, in doses separated by intervals of less than 3 weeks; and then
a therapeutically effective amount of said antibody or antibody derivative is administered to the patient for a second therapeutic period, in doses separated by intervals of at least 3 weeks.

The anti-CD40L antibody binds to CD40L on the surface of CD40L-expressing cells, such as activated T cells. In one embodiment, the anti-CD40L antibody is a monoclonal antibody. The monoclonal antibody may be 5c8 (ATCC Accession No. HB 10916).

The anti-CD40L antibody may be formulated in a therapeutic composition which includes a therapeutically-effective amount of the anti-CD4L antibody and a pharmaceutically acceptable carrier. The therapeutic composition may also include a second therapeutically effective compound.

### Brief Description of the Drawings

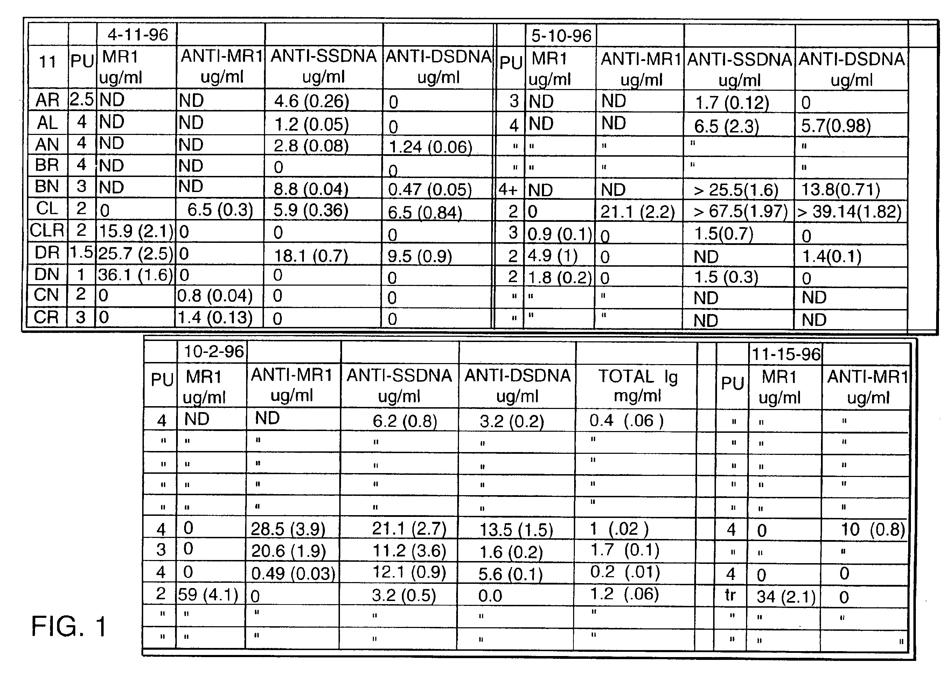

Fig. 1 is a chart of the changes with time in several measured characteristics of blood and urine from control and treated (SWR X NZB) F₁ mice in Experiment II. The anti-CD40L mAb MR1, at 500 µg/animal i.p., was administered once when the mice were 4 months old, again at 7 months of age, again at 9 months, and then at monthly intervals. Each of the upper five rows of the chart, marked AR-BN, contains data from a single control animal, and each of the lower six rows, marked CL-CR , contains data from a single treated animal. This study began when the animals were 4 months of age, in February 1996. The vertical double lines separate 4 groups of data, each data group providing the measurements for urine and blood samples collected on the date listed above the data. Proteinuria (PU) levels are indicated from trace to level 4. Level 1 correlates with urine albumin of 30 mg/dl albumin, level 2 with 100 mg/dl, level 3 with 300 mg/dl, and level 4 with over 2000 mg/dl. Levels of anti-MR1 antibodies (provided in column labeled Aanti-MR1 "), anti-ssDNA antibodies and anti-dsDNA antibodies are given in µg/ml blood. Where appropriate, values are given as mean and standard deviation of several samples, in the form mean(S.D.). A dash indicates that a sample was not collected, typically because the animal had died. ND refers to "not done."

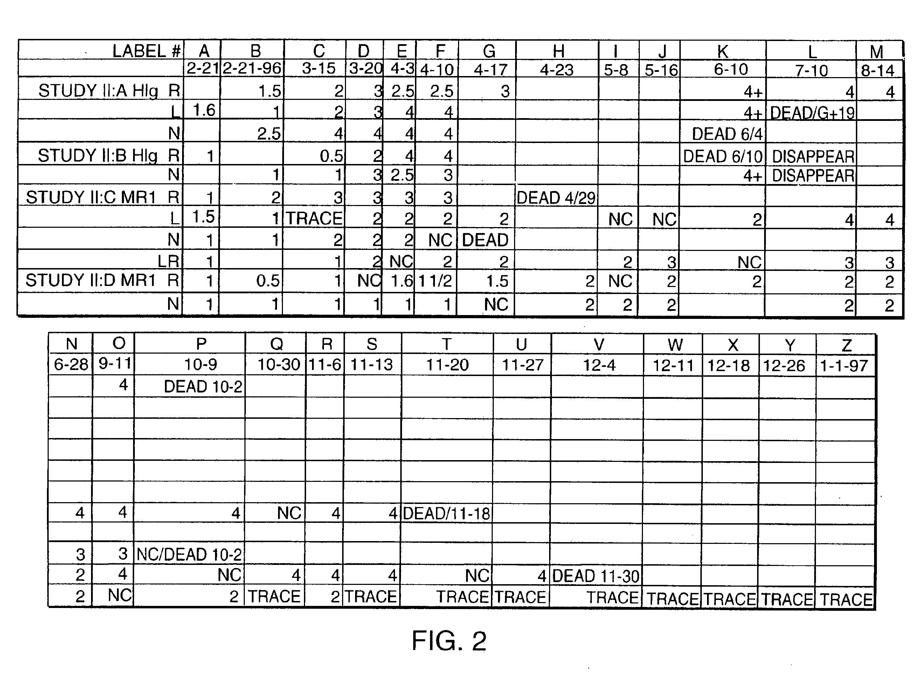

Fig. 2 is a chart of proteinuria measurements of the Experiment II animals over time. The first column provides the animal numbers as in Fig. 1. The columns are headed with the dates of sample collection. NC means Anot collected:@

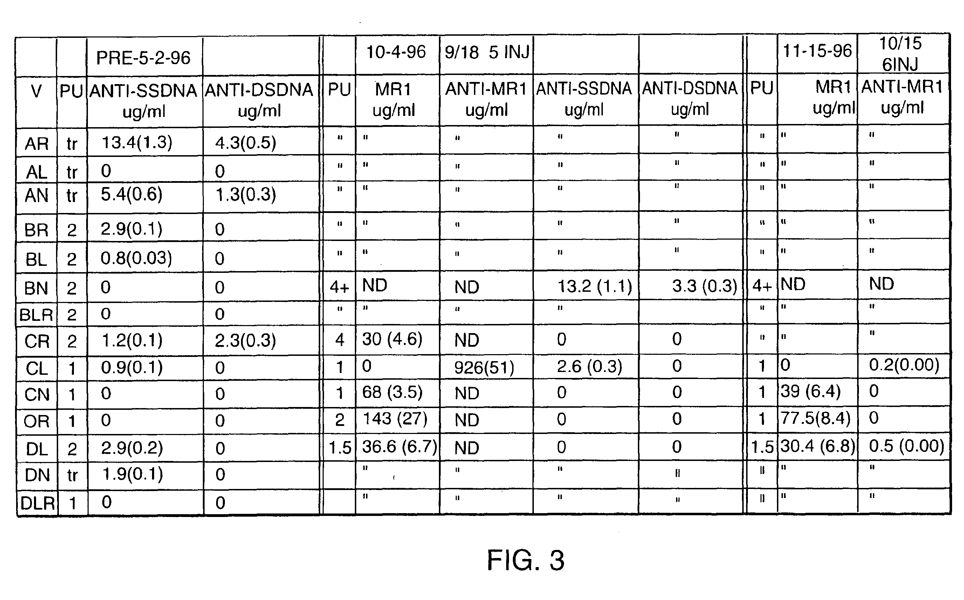

Fig. 3 is a chart of blood and urine characteristics with time in Experiment V control and untreated mice, which started treatment at 4.5 months of age. MR1 was administered to treated animals once at 500 µg/animal i.p. when the mice were 4.5 months old, and then as monthly injections of 500 µg, i.p. Each of the upper seven rows of the chart, marked AR-BLR, contains data from a single control animal, and each of the lower seven rows, marked CR -CLR, contains data from a single treated animal. This study began when the animals were 4.5 months of age, in May 1996. Other descriptions of the figure are the same as those of Fig. 1.

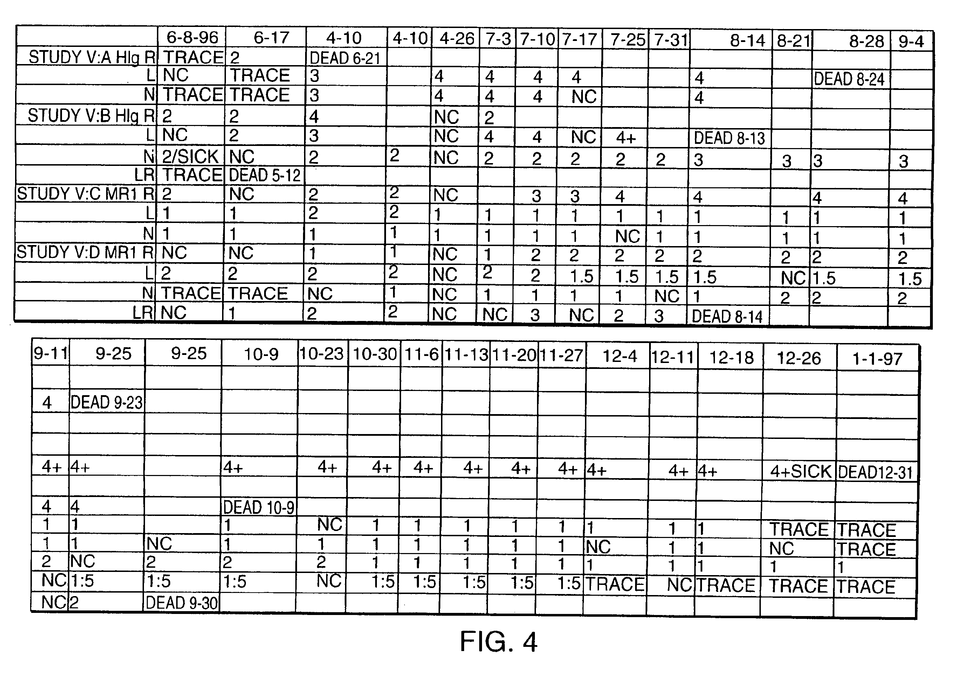

Fig. 4 is a chart of proteinuria measurements of the Experiment V animals over time. Animal numbers are as described for Fig. 3. Other descriptions of the figure are the same as those of Fig. 2.

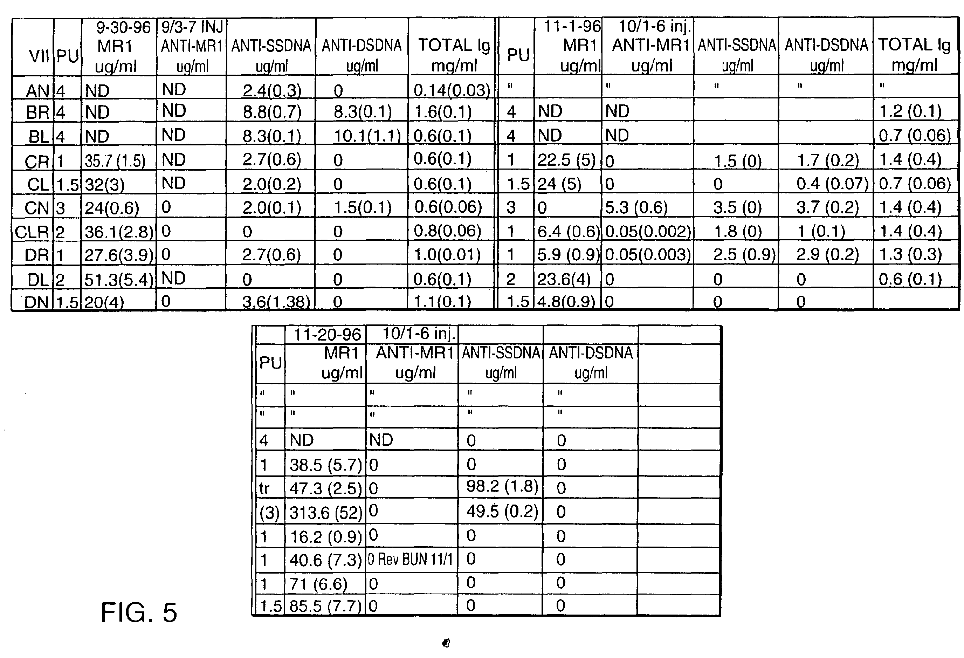

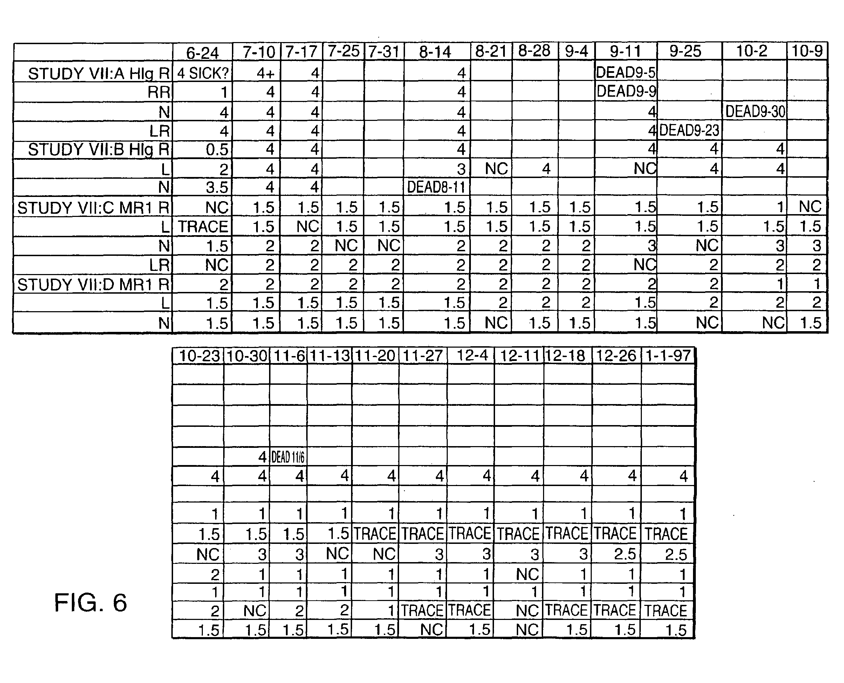

Fig. 5 is a chart of chart of blood and urine characteristics with time in Experiment VII control and untreated mice, which started treatment at 5.5 months of age. MR1 was administered to treated animals once weekly at 500 µg/animal i.p. for six weeks, followed by monthly injections of 500 µg, i.p. Each of the upper three rows of the chart, marked AN-BL, contains data from a single control animal (as noted in Fig. 6, some control animals had died before the data for Fig. 5 was collected), and each of the lower seven rows, marked CR-DN , contains data from a single treated animal. This study began when the animals were 5.5 months of age, in June 1996. Other descriptions of the figure are the same as those of Fig. 1.
Fig. 6 is a chart of proteinuria measurements of the Experiment VII animals over time. Each of the upper seven rows of the chart, marked AR-BN, contains data from a single control animal, and each of the lower seven rows, marked CR-DN , contains data from a single treated animal. Other descriptions of the figure are the same as those of Fig. 2.

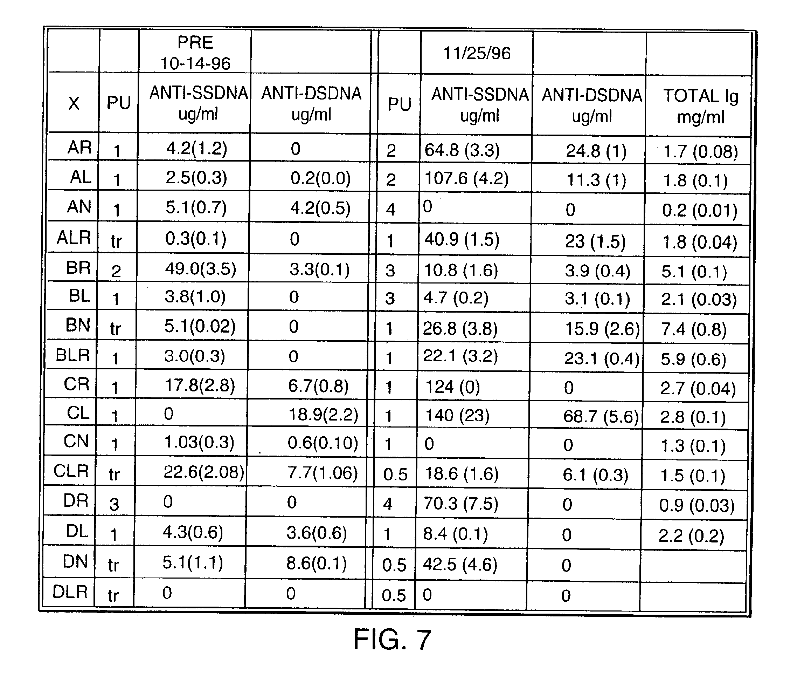

Fig. 7 is a chart of blood and urine characteristics with time in Experiment X control and untreated mice, which started treatment at 5.5 months of age. MR1 was administered to treated animals once weekly at 500 µg/animal i.p. for four weeks, followed by monthly injections of 200 µg, i.p. Each of the upper eight rows of the chart, marked AR-BLR, contains data from a single control animal, and each of the lower eight rows, marked CR-DLR , contains data from a single treated animal. This study began when the animals were 5.5 months of age, in October 1996. Other descriptions of the figure are the same as those of Fig. 1.

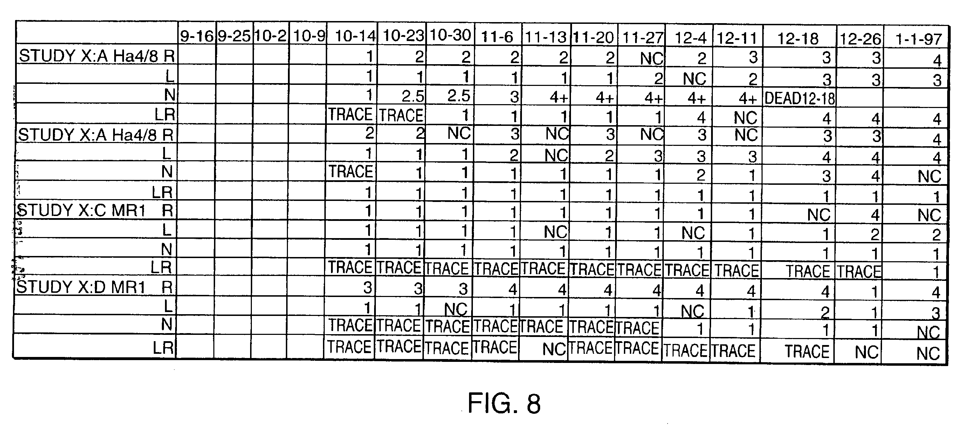

Fig. 8 is a chart of proteinuria measurements of the Experiment X animals over time. The first column provides the animal numbers as in Fig. 7. Other descriptions of the figure are the same as those of Fig. 2.

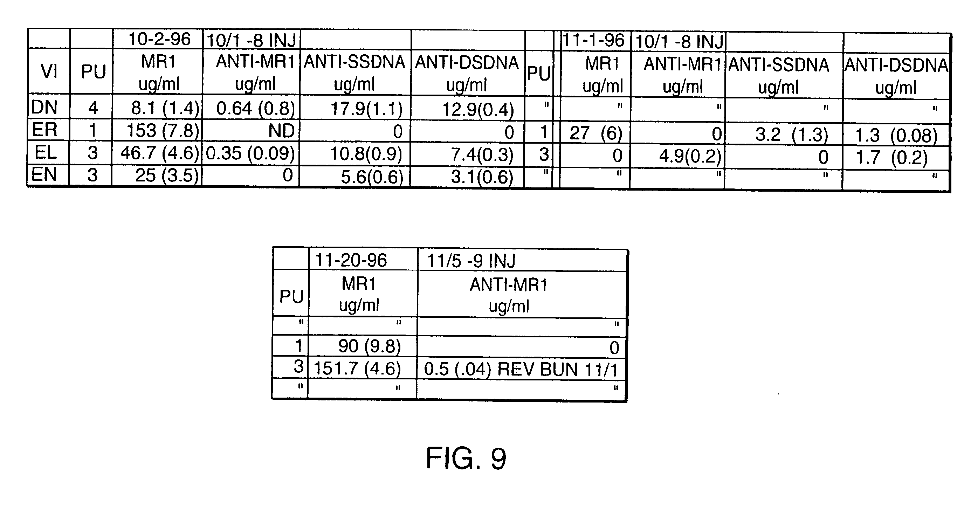

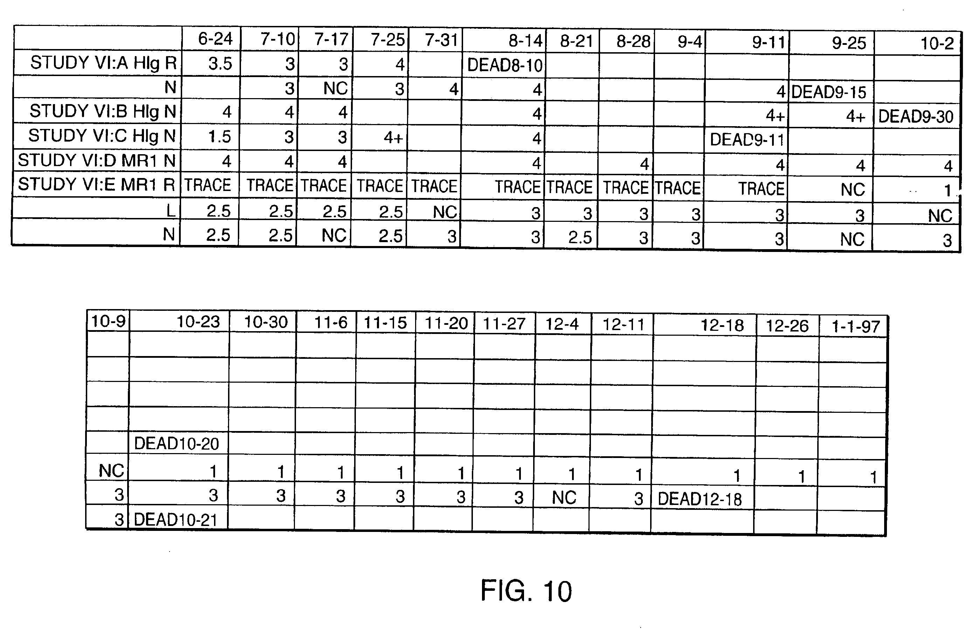

Fig. 9 is a chart of blood and urine characteristics with time in Experiment VI control and untreated mice, which started treatment at 7 months of age. MR1 was administered to 4 treated animals once weekly at 500 µg/animal i.p. for six weeks, followed by monthly injections of 500 µg, i.p. Each of the lower four rows, marked DN-EN, contains data from a single treated animal. At the time of first data collection for this chart, all control animals had died, as noted Fig. 10. This study began when the animals were 7 months of age, in June 1996. Other descriptions of the figure are the same as those of Fig. 1.
Fig. 10 is a chart of proteinuria measurements of the Experiment VI animals over time. Each of the upper four rows of the chart, marked AR-CN, contains data from a single control animal, and each of the lower four rows, marked DN-EN, contains data from a single treated animal. Other descriptions of the figure are the same as those of Fig. 2.

### Detailed Description of the Invention

The method described herein involves treating, preventing, reversing or stabilizing a patient with systemic lupus erythematosus, by treating the patient with an anti-CD40L antibody in therapeutic periods as outlined above. The antibody blocks the interaction of CD40L on T cells with CD40 on B cells, which is thought to inhibit the production of pathologic antibodies responsible for many of the pathologic effects of the disease.

### Compounds

Therapeutic compounds useful for the methods described herein include any anti-CD40L antibody that blocks the interaction of CD40 on B cells with CD40L expressed on the surface of activated T cells. Anti-CD40L compounds specifically contemplated include polyclonal antibodies and monoclonal antibodies (mAbs), as well as antibody derivatives such as chimeric molecules, humanized molecules, molecules with reduced effector functions, bispecific, molecules, and conjugates of antibodies. In a preferred embodiment, the antibody is 5c8, as described in U.S. Patent 5,474,771. Other known antibodies against 5c8 antigen include antibodies ImxM90, ImxM91 and ImxM92 (obtained from Immunex), an anti-CD40L mAb commercially available from Ancell (clone 24-31, catalog # 353-020, Bayport, MN), and an anti-CD40L mAb commercially available from Genzyme (Cambridge, MA, catalog # 80-3703-01). Also commercially available is an anti-CD40L mAb from PharMingen (San Diego, catalog #33580D). Numerous additional anti-CD40L antibodies have been produced and characterized (see, e.g., WO 96/23071 of Bristol-Myers Squibb).

The invention also includes the use of anti-CD40L molecules of other types, such as complete Fab fragments, F(ab')₂ compounds, V_{H} regions, Fᵥ regions or single chain antibodies (see, e.g., WO 96123071).

Various forms of antibodies may also be produced using standard recombinant DNA techniques (Winter and Milstein, Nature 349: 293-99, 1991). For example, "chimeric" antibodies may be constructed, in which the antigen binding domain from an animal antibody is linked to a human constant domain ( an antibody derived initially from a nonhuman mammal in which recombinant DNA technology has been used to replace all or part of the hinge and constant regions of the heavy chain and/or the constant region of the light chain, with corresponding regions from a human immunoglobin light chain or heavy chain) (see, e.g., Cabilly et al., United States patent 4,816,567; Morrison et al., Proc. Natl. Acad. Sci. 81: 6851-55, 1984). Chimeric antibodies reduce the immunogenic responses elicited by animal antibodies when used in human clinical treatments.

In addition, recombinant "humanized" antibodies may be synthesized. Humanized antibodies are antibodies initially derived from a nonhuman mammal in which recombinant DNA technology has been used to substitute some or all of the amino acids not required for antigen binding with amino acids from corresponding regions of a human immunoglobin light or heavy chain (chimeras comprising mostly human IgG sequences into which the regions responsible for specific antigen-binding have been inserted)(see, e.g., PCT patent application WO 94/04679). Animals are immunized with the desired antigen, the corresponding antibodies are isolated and the portion of the variable region sequences responsible for specific antigen binding are removed. The animal-derived antigen binding regions are then cloned into the appropriate position of the human antibody genes in which the antigen binding regions have been deleted. Humanized antibodies minimize the use of heterologous (interspecies) sequences in human antibodies and are less likely to elicit immune responses in the treated subject.

Also useful in the methods and compositions described herein are primate or primatized antibodies.

Antibody fragments and univalent antibodies may also be used in the methods and compositions described herein. Univalent antibodies comprise a heavy chain/light chain dimer bound to the Fc (or stem) region of a second heavy chain. "Fab region" refers to those portions of the chains which are roughly equivalent, or analogous, to the sequences which comprise the Y branch portions of the heavy chain and to the light chain in its entirety, and which collectively (in aggregates) have been shown to exhibit antibody activity. A F_{ab} protein includes aggregates of one heavy and one light chain (commonly known as Fab'), as well as tetramers which correspond to the two branch segments of the antibody Y, (commonly known as F(ab)₂), whether any of the above are covalently or non-covalently aggregated, so long as the aggregation is capable of selectively reacting with a particular antigen or antigen family.

In addition, standard recombinant DNA techniques can be used to alter the binding affinities of recombinant antibodies with their antigens by altering amino acid residues in the vicinity of the antigen binding sites. The antigen binding affinity of a humanized antibody may be increased by mutagenesis based on molecular modeling (Queen et al., Proc. Natl. Acad. Sci. 86:10029-33, 1989; PCT patent application WO 94/04679). It may be desirable to increase or to decrease the affinity of the antibodies for CD4OL, depending on the targeted tissue type or the particular treatment schedule envisioned. This may be done utilizing phage display technology (see, e.g., Winter et al./Ann. Rev. Immunol. 12:433-455, 1994; and Schier et al., J. Mol. Biol. 255:28-43, 1996. For example, it may be advantageous to treat a patient with constant levels of antibodies with reduced affinity for CD40L for semi-prophylactic treatments. Likewise, antibodies with increased affinity for CD40L may be advantageous for short-term treatments.

### Subjects

The term "patient" is taken to mean any patient to which anti-CD40L compounds may be administered. Patients specifically intended for treatment with the method described herein include humans.

The subjects for which the methods described herein are intended have systemic lupus erythematosus.

### Routes of Administration

The anti-CD40L antibodies employed in the invention may be administered in any manner which is medically acceptable. This may include injections, by parenteral routes Such as intravenous, intravascular, intraarterial, subcutaneous, intramuscular, intratumor, intraperitoneal, intraventricular, intraepidural, or others as well as oral, nasal, ophthalmic, rectal, or topical. Sustained release administration is also specifically included in the invention, by such means as depot injections. Some forms of anti-CD40L antibodies may be suitable for oral administration, and could be formulated as suspensions or pills.

### Dosages and Frequency of Treatment

The amount of dosing for any particular compound to be administered to a patient for a given immune complex disease is a judgment made by the patient's physician, based on a number of factors. The general dosage is established by preclinical and clinical trials, which involve extensive experiments to determine the beneficial and deleterious effects on the patient of different dosages of the compound. Even after such recommendations are made, the physician will often vary these dosages for different patients based on a variety of considerations, such as a patient's age, medical status, weight, sex, and concurrent treatment with other pharmaceuticals. Determining the optimal dosage for each anti-CD40L compound used to treat lupus nephritis is a routine matter for those of skill in the pharmaceutical and medical arts.

Various regimens may be used for treatment of lupus or other immune complex diseases according to this invention. Generally, the frequency of dosing would be determined by the attending physician, and might include periods of greater dosing frequency, such as at daily or weekly intervals, alternating with periods of less frequent dosing, such as at monthly or longer intervals.

To exemplify dosing considerations for an anti-CD40L antibody, the following examples of administration strategies are given for an anti-CD40L mAb. The dosing amounts could easily be adjusted for other types of anti-CD40L antibodies. In general, single dosages of between about 0.05 and about 50 mg/kg patient body weight are contemplated, with dosages most frequently in the 1-20 mg/kg range. For acute treatment, an effective dose of antibodies ranges from about 1 mg/kg body weight to about 20 mg/kg body weight, administered daily for a period of about 1 to 5 days, preferably by bolus intravenous administration. The same dosage and dosing schedule may be used in the load phase of a load-maintenance regimen, with the maintenance phase involving intravenous or intramuscular administration of antibodies in a range of about 0.1 mg/kg body weight to about 20 mg/kg body weight, for a treatment period of anywhere from weekly to 3 month intervals. Chronic treatment may also be carried out by a maintenance regimen, in which antibodies are administered by intravenous or intramuscular route, in a range of about 0.1 mg/kg body weight to about 20 mg/kg body weight, with interdose intervals being anywhere between about 1 week and about to 3 months. In addition, chronic treatment may be effected by an intermittent bolus intravenous regimen, in which between about 1.0 mg/kg body weight and about 100 mg/kg body weight of antibodies are administered, with the interval between successive treatments being from 1 to 6 months. For all except the intermittent bolus regimen, administration may also be by oral, pulmonary, nasal or subcutaneous routes.

Generally, therapy is commenced with low doses of antibodies. For example, an initial dose of antibodies is administered to the patient by, for example, infection or infusion. That initial dose should contain between about 1.0 mg and 30 mg of antibodies per day for a 70 kg patient. For repeated administrations over several days, dosages may be administered on successive days, every two to six days, once a week, every two to four weeks or once a month, until a desired suppression of disease symptoms is observed. However, other dosage regimens are also useful. When the symptoms have been alleviated to the desired level, treatment may cease. Patients may, however, require intermittent treatment on a long term basis upon recurrence of disease symptoms.

According to an alternate embodiment of this invention for treatment of lupus, the effectiveness of the antibodies may be increased by administration serially or in combination with conventional anti-lupus therapeutic agents or drugs such as, for example, salicylates, corticosteroids or immunosuppressants. Alternatively, the antibodies may be conjugated to a conventional agent. This advantageously permits the administration of the conventional agent in an amount less than the conventional dosage, for example, less than about 50% of the conventional dosage, when the agent is administered as monotherapy. Accordingly, the occurrence of many side effects associated with that agent might be avoided.

Combination therapies according to this invention for treatment of lupus include the use of anti-CD40L antibodies together with agents targeted at B cells, such as anti-CD19, anti-CD28 or anti-CD20 antibody (unconjugated or radiolabeled), IL-14 antagonists, LJP394 (LaJolla Pharmaceuticals receptor blocker), IR-1116 (Takeda small molecule) and anti-Ig idiotype monoclonal antibodies. Alternatively, the combinations may include T cell/B cell targeted agents, such as CTLA4Ig, IL-2 antagonists, IL-4 antagonists, IL-6 antagonists, receptor antagonists, anti-B7 monoclonal antibodies, TNF, LFA1/ICAM antagonists, VLA4/VCAM antagonists, brequinar and IL-2 toxin conjugates (e.g., DAB), prednisone, cyclophosphamide, and other immunosuppressants. Combinations may also include T cell targeted agents, such as CD4 antagonists, CD2 antagonists and IL-12.

Once improvement of the patient's condition has occurred, a maintenance dose of anti-CD40L antibodies, alone or in combination with a conventional anti-lupus agent is administered, if necessary. Subsequently, the dosage or the frequency of administration, or both, may be reduced, as a function of the symptoms, to a level at which the improved condition is retained. When the symptoms have been alleviated to the desired level, treatment might cease. In other instances, as determined by a patient's physician, occasional treatment might be administered, for example at intervals of four weeks or more. Patients may, however, require intermittent treatment on a long-term basis upon any recurrence of disease symptoms.

### Formulation

An anti-CD40L compound used in the methods of the invention is administered in a pharmaceutically-effective amount, which is an amount which produces a medically beneficial effect on a patient with an antibody-related disease, an immune-associated disorder, or a patient with a transplant or a transgene for which suppression of rejection is desirable.. Medically beneficial effects would include preventing deterioration or causing improvement in the patient's medical condition. As an example, an organ that is often damaged by pathologic antibodies is the kidney in SLE patients. In these patients, treated with the methods of the invention, renal function and health may be monitored with one or more laboratory tests which measure the concentrations of relevant substances in blood or urine, other urine characteristics, or the rate of clearance of various substances from the blood into the urine. The parameters measured by these tests, either individually or in combination, can be used by a physician to assess renal function or damage. Examples of such parameters include the blood concentration of urea, creatinine or protein; the urine concentration of protein or of various blood cells such as erythrocytes or leucocytes; urine specific gravity; amount of urine; the clearance rates of inulin, creatinine, urea or p-aminohippuric acid; and the presence of hypertension or edema. Medically beneficial effects would also include the diminution of autoantibodies, such as anti-dsDNA antibodies in the serum of lupus patients.

An anti-CD40L antibody is administered to a patient in a pharmaceutically acceptable composition, which may include a pharmaceutically-acceptable carrier. Such a carrier is relatively non-toxic and innocuous to a patient at concentrations consistent with effective activity of the anti-CD40L antibody or other active ingredients, so that any side effects ascribable to the carrier do not vitiate the beneficial effects of the active ingredients of the composition. The composition may include other compatible substances; compatible, as used herein, means that the components of the pharmaceutical composition are capable of being commingled with the anti-CD40L antibody, and with each other, in a manner such that there is no interaction which would substantially reduce the therapeutic efficacy of the pharmaceutical. Nasal spray formulations comprise purified aqueous solutions of the active compound with preservative agents and isotonic agents. Such formulations are preferably adjusted to a pH and isotonic state compatible with the nasal mucous membranes. Formulations of the present invention suitable for oral administration may be presented as discrete units such as capsules, cachets, tablets, pills or lozenges, each containing a predetermined amount of the potentiating anti-CD40L antibody as a powder or granules; as liposomes; or as a suspension in an aqueous liquor or non-aqueous liquid such as a syrup, an elixir, an emulsion or a draught.

### Use of Anti-CD40L Compounds Administered at Wide Intervals to Treat Lupus Nephritis in Nonhuman Subjects

We chose to demonstrate the efficacy of administering anti-CD40L compounds in an animal model of lupus nephritis. Systemic lupus erythematosus (SLE) is a life threatening 5 autoimmune disease, characterized by the production of autoantibodies against various tissues, and often against DNA. SLE affects approximately 140,000 people in the United States and 105,000 in western Europe, predominantly women of childbearing age. In most patients, lupus-associated immunoglobulins and immune complexes are deposited in the renal glomeruli, causing a decline in renal function. If widely spaced doses of anti-CD40L 10compounds are efficacious in selectively suppressing antibody production, such a dosing regime would exert beneficial effects on nephritis. This could be evidenced in treated animals by slower progression of nephritis, reduced severity of nephritis, enhanced survival, or even by improvement of renal function in some animals.

We tested the effects of the hamster anti-muCD40L mAb MR1 on the course of 15nephritis in the female (SWR X NZB) F₁ mouse, in several studies as described below. Control animals were injected either with Syrian hamster polyclonal Ig or with Ha4/8, an Armenian hamster mAb directed against KLH. Proteinuria levels are indicated from trace to level 4. Level 1 correlates with urine albumin of 30 mg/dl albumin, level 2 with 100 mg/dl, level 3 with 300 mg/dl, and level 4 with over 2000 mg/dl. A level of 2 was considered to 20indicate moderate nephritis, with 2.5 and greater indicating severe nephritis.

If untreated, or if treated with the nonspecific hamster immunoglobulins administered to control animals, the mice normally die by 12 months of age. While the onset of proteinuria in untreated animals is variable, most have mild to moderate proteinuria by 3 months of age; the proteinuria tends to increase with age. By about 5 months of age, all control animals 25 typically have detectable anti-dsDNA antibodies, and most have detectable anti-ssDNA antibodies; this contrasts with the complete lack of detectable levels of these antibodies in normal mice, such as the female SWR parents of the (SWR X NZB) F₁ mice.

### Experiment II: Treatment begun at 4 months (Figs. 1 and 2)

MR1 treatment was initiated when the mice were 4 months of age. MR1 was administered to treated animals once at 500 µg/animal i.p. when the mice were 4 months old, once at 7 months of age, and once at 9 months followed by once-monthly injections. After 4 months of treatment, 4 of the 5 control animals had died, but four of the six treated animals were yet alive. Three of these four previously surviving treated mice died, one each at 12, 13 and 13.5 months. One still survives, and is now 15 months old, an extraordinary longevity for mice of this cross. Of great interest, the surviving animal (mouse II:DN on Figure 2) had moderate nephritis (2+ proteinuria) from ages 8 to 13 months, which has decreased to only 10trace levels of proteinuria for the last two months. This is the first demonstration of a functional reversal of nephritis in a mouse of this strain.

### Experiment V: Treatment begun at 4.5 months (Figs. 3 and 4)

MR1 treatment was initiated when the mice were 4.5 months of age. MR1 was administered to treated animals once at 500 µg/animal i.p. when the mice were 4.5 months old, and then as monthly injections of 500 µg, i.p. After 4.5 months, 6 of the 7 control animals had died, but six of the seven treated animals survived. After 8 months of treatment, all controls were dead, but only three of the seven treated mice had died. As shown in Fig. 4, four of the seven MR1-treated animals had their nephritis reversed as shown by sustained 20lowered proteinuria levels. These four animals are still alive at age 12.5 months.

### Experiment VII: Treatment begun at 5.5 months (Figs. 5 and 6)

MR1 treatment was initiated when the mice were 5.5 months of age. MR1 at 500 µg/animal i.p. was administered to treated animals once weekly for six weeks, followed by monthly injections. After 5 months of treatment, at age 10.5 months, 6 of the 7 control animals had died; all of the 7 treated animals are still alive at age 12 months. The following values were measured in the animals which still survived at 8.5 months, after 3.5 months of treatment:

| | anti-SS-DNA | anti-DS DNA | PU |
|---|---|---|---|
| control | 2.4 | 0 | 4 |
| | 8.8 | 6.3 | 4 |
| | 6.3 | 10.1 | 4 |
| Mean (Std. Dev.) | 5.8 (2.6) | 5.4 (4.1) | 4 (0) |
| | | | |
| MR1 | 2.7 | 0 | 1 |
| | 2.0 | 0 | 1.5 |
| | 2.0 | 1.5 | 3 |
| | 0 | 0 | 2 |
| | 2.7 | 0 | 1 |
| | 0 | 0 | 2 |
| | 3.5 | 0 | 1.5 |
| Mean (Std. Dev.) | 1.8 (1.2) | 0.2 (0.5) | 1.7 ( ) |

### Experiment X: Less intensive treatment begun at 5.5 months (Figs. 7 and 8)

MR1 treatment was initiated when the mice were 5.5 months of age. MR1 was administered to treated animals once weekly at 500 µg/animal i.p. for four weeks, followed by monthly injections of 200µg, i.p. Of the 16 mice in the study (8 each in control and treated groups), now 8.5 months of age, only one mouse has died, a control animal at age 7.5 months. As shown in Fig. 8, seven of the eight control animals had proteinuria which steadily increased to high levels, averaging +3.4 for the 7 surviving control mice. All but one of the eight MR1- treated mice have maintained low proteinuria, which currently averages +2 for the 8 treated mice. As shown in Fig. 7, six of the treated animals, but only one of the controls, have no detectable anti-dsDNA antibodies.

### Experiment VI: Treatment begun at 7 months (Figs. 9 and 10)

MR1 treatment was initiated when the mice were seven months of age. MR1 was administered to 4 treated animals once weekly at 500 µg/animal i.p. for six weeks, followed by monthly injections of 500 µg, i.p. By age 10 months, all 4 control animals had died. While 2 of the treated mice died at age 11 months, and a third at 13 months, one of the four treated animals remains alive currently at 14 months of age, after 7 months of treatment. The surviving treated animal (number VI:ER) currently has level 1 proteinuria, and detectable anti-dsDNA and anti-ssDNA antibodies.

These experiments show that treatment of (SWR X NZB) F₁ mice with anti-CD40L mAb, administered for at least a period of time at intervals of over 3 weeks, markedly and consistently prolongs survival as compared to control animals, and slows development of nephritis as indicated by proteinuria levels. In some animals, the treatment actually reverses nephritis, as shown by a reduction in proteinuria levels. Of 32 treated animals, 11 had urine protein levels which decreased with anti-CD40L mAb therapy; none of the control animals had similar reductions. Of 24 treated animals in which serum blood urea nitrogen (BUN) was measured, 3 had decreases in BUN levels after treatment, which was not observed in any control animal. In addition, MR1 treatment often results in a reduced serum concentration of anti-DS and anti-SS DNA autoantibodies, which are normally produced in untreated animals of this type.

The disease-reducing or -preventing results of these experiments demonstrate that anti-CD40L compounds may successfully be used to treat antibody-associated conditions when administered at intervals of 3 or more weeks. This is a surprising and unanticipated finding, which confers significant advantages over previously contemplated dosing regimens. Particularly for patients being treated for a chronic disease, reduced frequency of treatments results in lowered cost, inconvenience, and discomfort, particularly for injectable or intravenous treatments. In addition, any side effects of treatment would be expected to be reduced with fewer and more widely spaced dosings.

Although the foregoing invention has been described in some detail by way of illustration and example for purposes of clarity of understanding, it will be obvious to one skilled in the art that certain changes and modifications may be practiced within the scope of the invention, as limited only by the scope of the appended claims.

## Claims

1. Use of an anti-CD40L antibody or an anti-CD40L antibody derivative for the manufacture of a medicament for treating a human patient with systemic lupus erythematosus, wherein:
a therapeutically effective amount of said antibody or antibody derivative is administered to the patient for a first therapeutic period, in doses separated by intervals of less than 3 weeks; and then
a therapeutically effective amount of said antibody or antibody derivative is administered to the patient for a second therapeutic period, in doses separated by intervals of at least 3 weeks.

2. The use according to claim 1, wherein said anti-CD40L antibody is a monoclonal antibody.

3. The use according to claim 1 or 2, wherein said anti-CD40L antibody is a humanized anti-CD40L antibody.

4. The use according to any one of claims 1 to 3, wherein said anti-CD40L antibody is an antibody against 5c8 antigen.

5. The use according to claim 1 or 2, wherein said anti-CD40L antibody is monoclonal antibody 5c8 obtainable from the deposited hybridoma ATCC Accession No: HB10916.

6. The use according to claim 1, wherein said anti-CD40L antibody derivative is selected from: Fab fragments, F (ab')₂ fragments, V_{H} regions and single chain antibodies.

7. The use according to any one of claims 1 to 6,
wherein, during said first therapeutic period:
(a) doses are separated by weekly intervals; or
(b) doses are separated by intervals of 2 to 6 days; or
(c) doses are separated by daily intervals.

8. The use according to any one of claims 1 to 6, wherein; during said second therapeutic period, doses are separated by intervals of at least 4 weeks.

9. The use according to any one of claims 1 to 6, wherein the therapeutically effective amount of said anti-CD40L antibody or antibody derivative is an amount sufficient to:
(a) prevent deterioration of the patient's medical condition; or
(b) cause improvement in the patient's medical condition.

10. The use according to any one of claims 1 to 6, wherein the therapeutically effective amount of said anti-CD40L antibody or antibody derivative is administered to the patient for the first therapeutic period in at least four doses separated by weekly intervals; and then a therapeutically effective amount of said anti-CD40L antibody or antibody derivative is administered to the patient for the second therapeutic period in doses separated by monthly intervals.

11. The use according to claim 10, wherein during the first therapeutic period, said anti-CD40L antibody or antibody derivative is administered to the patient in 6 doses.

## Patentansprüche

1. Verwendung eines Anti-CD40L-Antikörpers oder eines Anti-CD40L-Antikörperderivats für die Herstellung eines Medikamentes zum Behandeln eines menschlichen Patienten mit systemischem Lupus erythematosus, wobei
dem Patienten eine therapeutisch wirksame Menge des Antikörpers oder Antikörperderivates für einen ersten therapeutischen Zeitraum in Dosen verabreicht wird, die durch Intervalle von weniger als drei Wochen getrennt sind, und dann
dem Patienten eine therapeutisch wirksame Menge des Antikörpers oder Antikörperderivats für einen zweiten therapeutischen Zeitraum in Dosen verabreicht wird, die durch Intervalle von mindestens drei Wochen getrennt sind.

2. Verwendung gemäß Anspruch 1, wobei der Anti-CD40L-Antikörper ein monoklonaler Antikörper ist.

3. Verwendung gemäß Anspruch 1 oder 2, wobei der Anti-CD40L-Antikörper ein humanisierter Anti-CD40L-Antikörper ist.

4. Verwendungen gemäß einem der Ansprüche 1 bis 3, wobei der Anti-CD40L-Antikörper ein Antikörper gegen das Antigen 5c8 ist.

5. Verwendung gemäß Anspruch 1 oder 2, wobei der Anti-CD40L-Antikörper der monoklonale Antikörper 5c8 ist, der aus dem hinterlegten Hybridom mit der ATCC-Zugangsnummer HB10916 erhältlich ist.

6. Verwendung gemäß Anspruch 1, wobei das Anti-CD40L-Antikörperderivat ausgewählt ist aus Fab-Fragmenten, F(ab')₂-Fragmenten, V_{H}-Regionen und Einzelkettenantikörpern.

7. Verwendung gemäß einem der Ansprüche 1 bis 6, wobei während des ersten therapeutischen Zeitraums
a) die Dosen durch wöchentliche Intervalle getrennt sind, oder
b) die Dosen durch Intervalle von zwei bis sechs Tagen getrennt sind, oder
c) die Dosen durch tägliche Intervalle getrennt sind.

8. Verwendung gemäß einem der Ansprüche 1 bis 6, wobei während des zweiten therapeutischen Zeitraums die Dosen durch Intervalle von mindestens vier Wochen getrennt sind.

9. Verwendung gemäß einem der Ansprüche 1 bis 6, wobei die therapeutisch wirksame Menge des Anti-CD40L-Antikörpers oder des Anti-CD40L-Antikörperderivates eine Menge ist, die ausreicht, um
a) eine Verschlechterung des medizinischen Zustandes des Patienten zu verhindern, oder
b) eine Verbesserung des medizinischen Zustandes des Patienten zu verursachen.

10. Verwendung gemäß einem der Ansprüche 1 bis 6, wobei die therapeutisch wirksame Menge des Anti-CD40L-Antikörpers oder des Anti-CD40L-Antikörperderivates dem Patienten für den ersten therapeutischen Zeitraum in mindestens vier Dosen verabreicht wird, die durch wöchentliche Intervalle getrennt sind, und dann eine therapeutisch wirksame Menge des Anti-CD40L-Antikörpers oder des Anti-CD40L-Antikörperderivates dem Patienten für den zweiten therapeutischen Zeitraum in Dosen verabreicht wird, die durch monatliche Intervalle getrennt sind.

11. Verwendung gemäß Anspruch 10, wobei während des ersten therapeutischen Zeitraums der Anti-CD40L-Antikörper oder das Anti-CD40L-Antikörperderivat dem Patienten in sechs Dosen verabreicht wird.

## Revendications

1. Utilisation d'un anticorps anti-CD40L ou d'un dérivé d'anticorps anti-CD40L pour la fabrication d'un médicament pour traiter un patient humain atteint de lupus érythémateux systémique, dans laquelle:
une quantité efficace sur le plan thérapeutique dudit anticorps ou dérivé d'anticorps est administrée au patient pendant une première période thérapeutique, à des doses séparées par des intervalles de moins de 3 semaines; puis
une quantité efficace sur le plan thérapeutique dudit anticorps ou dérivé d'anticorps est administrée au patient pendant une seconde période thérapeutique, à des doses séparées par des intervalles d'au moins 3 semaines.

2. Utilisation selon la revendication 1, dans laquelle ledit anticorps anti-CD40L est un anticorps anti-CD40L monoclonal.

3. Utilisation selon la revendication 1 ou la revendication 2, dans laquelle ledit anticorps anti-CD40L est un anticorps anti-CD40L humanisé.

4. Utilisation selon l'une quelconque des revendications 1 à 3, dans laquelle ledit anticorps anti-CD40L est un anticorps dirigé contre l'antigène 5c8.

5. Utilisation selon la revendication 1 ou la revendication 2, dans laquelle ledit anticorps anti-CD40L est un anticorps monoclonal 5c8 pouvant être obtenu à partir de l'hybridome déposé portant le N° d'accès ATCC HB10916.

6. Utilisation selon la revendication 1, dans laquelle ledit dérivé de l'anticorps anti-CD40L est sélectionné parmi: des fragments Fab, des fragments F(ab')₂, des régions V_{H} et des anticorps à chaîne unique.

7. Utilisation selon l'une quelconque des revendications 1 à 6, dans laquelle, pendant ladite première période thérapeutique:
(a) les doses sont séparées par des intervalles d'une semaine; ou
(b) les doses sont séparées par des intervalles de 2 à 6 jours; ou
(c) les doses sont séparées par des intervalles d'un jour.

8. Utilisation selon l'une quelconque des revendications 1 à 6, dans laquelle, pendant ladite seconde période thérapeutique, les doses sont séparées par des intervalles d'au moins quatre semaines.

9. Utilisation selon l'une quelconque des revendications 1 à 6, dans laquelle la quantité efficace sur le plan thérapeutique dudit anticorps anti-CD40L ou dudit dérivé d'anticorps est une quantité suffisante pour:
(a) prévenir la détérioration de l'état de santé du patient; ou
(b) produire une amélioration de l'état de santé du patient.

10. Utilisation selon l'une quelconque des revendications 1 à 6, dans laquelle la quantité efficace sur le plan thérapeutique dudit anticorps anti-CD40L ou du dérivé d'anticorps est administrée au patient pendant la première période thérapeutique en au moins quatre doses séparées par des intervalles d'une semaine; puis une quantité efficace sur le plan thérapeutique dudit anticorps anti-CD40L ou du dérivé d'anticorps est administrée au patient pendant la seconde période thérapeutique à des doses séparées par des intervalles d'un mois.

11. Utilisation selon la revendication 10, dans laquelle, pendant la première période thérapeutique, ledit anticorps anti-CD40L ou le dérivé d'anticorps sont administrés au patient en six doses.
